# EUROPEAN PATENT SPECIFICATION

(11) **EP 3 566 695 B1**
(45) Date of publication and mention of the grant of the patent: **05.08.2020**
(21) Application number: 19180399.8
(22) Date of filing: 06.12.2013
(51) Int. Cl.: A61K 9/08, A61K 9/10, A61K 31/53, A61K 33/26, A61P 33/02, A61K 31/7135, A61K 45/06

(54) **TREATMENT OF COCCIDIOSIS WITH INTRAMUSCULAR TRIAZINE COMPOSITIONS**
BEHANDLUNG VON KOKZIDIOSE MIT INTRAMUSKULÄREN TRIAZINZUSAMMENSETZUNGEN
TRAITEMENT DE LA COCCIDIOSE AVEC DES COMPOSITIONS DE TRIAZINE INTRAMUSCULAIRES

(30) Priority: 07.12.2012 EP 12306547
(43) Date of publication of application: 13.11.2019
(62) Divisional of application: 13815401.8
(73) Proprietor: Ceva Santé Animale, 33500 Libourne (FR)
(72) Inventor: KAREMBE, Hamadi, 33500 LIBOURNE (FR); KREJCI, Roman, 33500 LIBOURNE (FR); GUYONNET, Jérôme, 33500 LIBOURNE (FR); CILLIERS, Hannelie, 33500 LIBOURNE (FR)
(74) Representative: Hoyng Rokh Monegier LLP

(56) References cited:
- WO-A1-02/14288
- DE-A1-102007 025 908
- DE-A1-102009 038 949
- US-B1- 6 465 460
- -: "Baycox (R) 5% Toltrazuril - Coccidiocide for Piglets - Technical Information", BAYER, January 2003 (2003-01), pages 1-31, XP55453605,
- PAN B L ET AL: "Effect of subcutaneously administered diclazuril on the output of Eimeria species oocysts by experimentally infected rabbits", VETERINARY RECORD, BRITISH VETERINARY ASSOCIATION, LONDON, GB, vol. 162, no. 5, 2 February 2008 (2008-02-02), pages 153-155, XP009166529, ISSN: 0042-4900, DOI: 10.1136/VR.162.5.153

## Description

The invention relates to intramuscular compositions for treating coccidiosis with toltrazuril.

### Introduction

Triazines are commonly used in the veterinary industry to treat non-human mammals against a variety of diseases. Triazines are broad-spectrum antimicrobials that inhibit both gram positive and gram-negative bacteria, as well as some protozoa, such as coccidia.

Coccidiosis is a parasitic disease of the intestinal tract of animals, caused by coccidian protozoa such as e.g., *Eimeria* or *Isospora.* The disease spreads from one animal to another by contact with infected feces or ingestion of infected tissue. Diarrhea, which may become bloody in severe cases, is the primary symptom. Young animals such as piglets suffer severe symptoms, which may ultimately lead to death.

Triazines, such as toltrazuril and ponazuril, are authorized agents for the treatment and control of coccidiosis. They are essentially administered to the animals by oral route. In this regard, EP116175 refers to a water miscible solution of a triazine for oral administration. DE19603984 proposes triazine granules for oral administration and DE19824483 relates to semi-solid preparations which are applied orally. EP2164496 relates to triazine-iron combination products. The triazine compound is administered orally, as a suspension.

Administration of triazines by transdermal application has been proposed in US2010/0179151. Transdermal application as proposed in US2010/0179151 comprises a spot on formulation which is applied on the skin and taken up by passive percutaneous absorption. Such administration route does not allow a strict control of the dosage administered to each animal. Also, transdermal administration cannot be combined with other treatments which are given by injection (e.g., antibiotics, anti -inflammatory agents, anthelmintics, endectocides, minerals such as iron, or vitamins).

WO01/26660 and US6,465,460 relate to a sodium salt of triazine compounds and to compositions for oral or parenteral administration. According to this patent the sodium salt allows the use of lower doses of the compound. Although different administration routes are mentioned, all of the experimental section is limited to intravenous and oral administration. Furthermore, the patent indicates that several administrations and/or a sustained release dose are required for maintaining appropriate blood levels. In particular, the patent proposes a starting dose and several maintenance doses, which need to be administered over several days.

Despite preliminary investigations on different routes (e.g., transdermal), oral administration is still preferred today because it is believed to provide the most appropriate pharmacokinetic profile of the drug, especially in young animals. In particular, while the oral formulation requires substantive manipulation and cannot provide a strict dosage control, it is believed to ensure appropriate bioavailability and therapeutic efficacy of the triazine compounds.

Upon infection of animals with the causative protozoan (usually within the first week of life), the first symptoms of the disease usually appear 3-5 days post-infection. Accordingly, to obtain best protection of the animals, an anti-microbial effect is required within 5 days post birth. When triazines such as toltrazuril are administered orally between days 3 to 5 after birth, a peak of toltrazuril-sulfone (i.e ponazuril), an active metabolite, may be reached in the blood at day 5, which provides a protection against the disease.

Oral administration, however, presents drawbacks. Indeed, oral administration prevents strict control of the dosage administered to each animal. Also, oral administration cannot be conveniently combined with other treatments which are given by injection (e.g., antibiotics, anti-inflammatory agents, anthelmintics, endectocides, minerals such as iron, or vitamins) at newborn to 3 days old piglets and therefore involves additional manipulation of the animals.

Such drawbacks have not been overcome in the art since alternative routes e.g., transdermal application, also have the same limitations.

It has now been surprisingly found that triazines, in particular toltrazuril may be administered by intramuscular injection. Surprisingly, the inventors have found that full activity can be achieved with a single intramuscular injection of triazines, in particular toltrazuril, and that such administration in young piglets (newborn to 3 days old) can provide the required serum concentrations and release schedule of the active agent to protect the animal against infections with protozoans such as coccidia. In particular, although the intramuscular application modifies the pharmacokinetic profile of toltrazuril itself, it has now been surprisingly found that such application leads to an optimized pharmacokinetic profile of an active metabolite thereof, toltrazuril-sulfone (i.e ponazuril). The invention thus allows an efficient administration of toltrazuril, even at very early stages of growth of the pig. In addition, the invention allows a combined administration of toltrazuril with other agents that are administered early by injection, such as antibiotics, anti-inflammatory agents, anthelmintics, endectocides, minerals such as iron, or vitamins. Furthermore, contrary to the usual knowledge, the inventors have found that the combined intramuscular injection of toltrazuril, and iron complex to one-day piglets did not cause particular stress and represents a very efficient approach for treating young animals.

### Summary of the invention

An object of the invention resides in a composition comprising toltrazuril, for use in treating coccidiosis or a related disease in a a piglet, wherein said composition is administered by intramuscular injection.

The compositions of the invention are administered by a single intramuscular injection.

The compositions of the invention are administered to newborn to 3 days old animals, preferably newborn to 48 hours after birth.

The compositions of the invention are preferably administered in a muscle of the neck or behind the ear.

Preferred compositions of the invention comprise toltrazuril and an iron complex.

The invention may be used to treat (e.g., prevent, retard, protect against, reduce or cure) coccidiosis. In a particular embodiment, the invention is used for the preventive treatment of coccidiosis, e.g., to protect the animal against onset or development of the disease.

The invention is adapted for the treatment of newborn to 3 days pigs, more preferably newborn to 48 hours.

### Legend to the Figures

Fig 1: Local reaction Score of treated and non-treated piglets G1: Intramuscular 20 mg/kg by bodyweight (0.4 ml/kg); G2: Intramuscular 60 mg/kg by bodyweight (1.2 ml/kg).
Fig 2: Average weight gain of treated and non-treated piglets: G1: Intramuscular 20 mg/kg by bodyweight (0.4 ml/kg); G2: Intramuscular 60 mg/kg by bodyweight (1.2 ml/kg); G3: Oral 20 mg/kg by bodyweight (0.4 ml/kg); G4: Control, not treated;
Fig 3: Fecal consistency of treated and non-treated piglets.
Fig 4: Bodyweight change of treated and non-treated piglets following intramuscular injection of 20 mg/kg by bodyweight (Group 1); intramuscular injection of 40 mg/kg by bodyweight (Group2); oral administration of 20 mg/kg by bodyweight (Group 3) or non-treated control (Group 4)
Fig 5 : Mean PK profile of toltrazuril and toltrazuril-sulfone following intramuscular injection of 20mg/kg by bodyweight (A) or 40mg/kg by bodyweight (B) toltrazuril, or following oral administration of 20 mg/kg by bodyweight toltrazuril (C).

### Detailed description of the invention

The invention resides in compositions for use as defined in the claims.

### Triazine compounds

Toltrazuril (1-methyl-3-[3-methyl-4-[4-(trifluoromethyl)tio)phenoxil]phenyl]-1,3,5-triazine(1H,3H,5H)-2,4,6-trione) is widely used in porcine, ovine, bovine and avian for the prevention and treatment of coccidiosis, by oral administration. It is currently available on the market as Cevazuril^{R} or Baycox^{R}. Methods for the preparation of toltrazuril are disclosed in various patents such as US 4,219,552, US 5,219,853, EP 0 201 030, and EP 0 879 057. The chemical structure of toltrazuril is represented in formula (C) below:

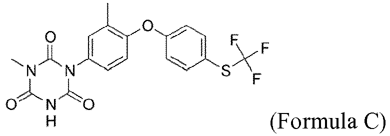

### Treatment

Within the context of the disclosure, the term treatment includes, particularly, the preventive treatment of non-human animals against a disease. The preventive treatment of an animal against a disease designates a treatment made before the animal has been exposed to or in contact with the causative agent of the disease (e.g., a pathogen, virus, protozoan, cell, etc.), or after said exposure/contact but before development of the symptoms or at an early stage of development of the disease. Also, the term preventive treatment, in relation to a population of animals, designates the treatment of all members of the population even after the disease has been detected in certain members, to limit or avoid spreading of and contamination to the others.

In a particular embodiment, the term treatment designates the protection of an animal against a disease, e.g., against the effect of an exposure to the causative agent, or against the development of the disease in exposed animals. The invention is particularly suited to protect pigs against coccidiosis.

The term treatment also includes an increase in the welfare of the treated animals, for example in increasing the production of meat.

The term treatment or preventive treatment also includes the alleviation of the symptoms, as well as a delay, reduction or cure of an existing infection.

### Coccidiosis

The term "coccidiosis" includes any disease caused by the coccidian protozoa *Isospora.* Coccidiosis typically manifests by diarrhea and/or fever. The disease can spread from one animal to another by contact with infected feces or ingestion of infected tissue.

For example in piglets, the predominant sign of coccidiosis is diarrhea, which usually persists for 4 to 6 days. The faeces may vary from white to yellow in color and from a fluid to a pasty consistency, usually without the presence of blood. Coccidiosis predisposes the piglet to the incidence of secondary bacterial infections and severely affected piglets may die.

Although the morbidity is usually high, mortality is variable, probably due to the difference in the number of oocysts ingested, differences in the environment and the presence of other co-existing disease problems. Although the disease has only a slight influence on mortality, it does influence the presence of concomitant infections and the amount of antibiotics needed to control them. The disease also drastically affects the development of piglets, where the daily weight gain of the infected animals decreases compared to uninfected animals, causing herds to have poor uniformity at weaning age. There is notable emaciation and stunting. Coccidiosis reduces growth by about 15% on average, that is, minimum 500g at weaning age, and this contributes to weaning herds that are highly heterogenous

Examples of economically important coccidioses are: infections of pigs with coccidia of the genus *Isospora.*

The invention is effective against all stages of development of the pathogen.

The term "anemia" includes any iron deficiency.

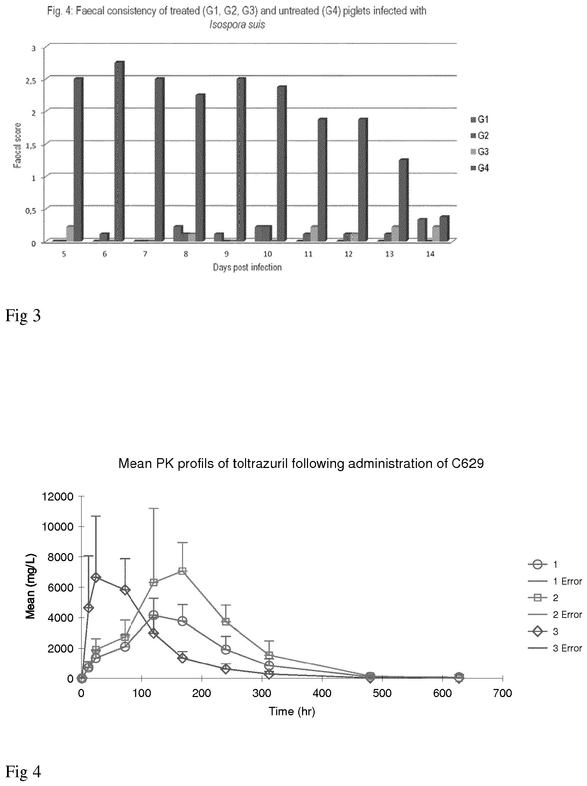

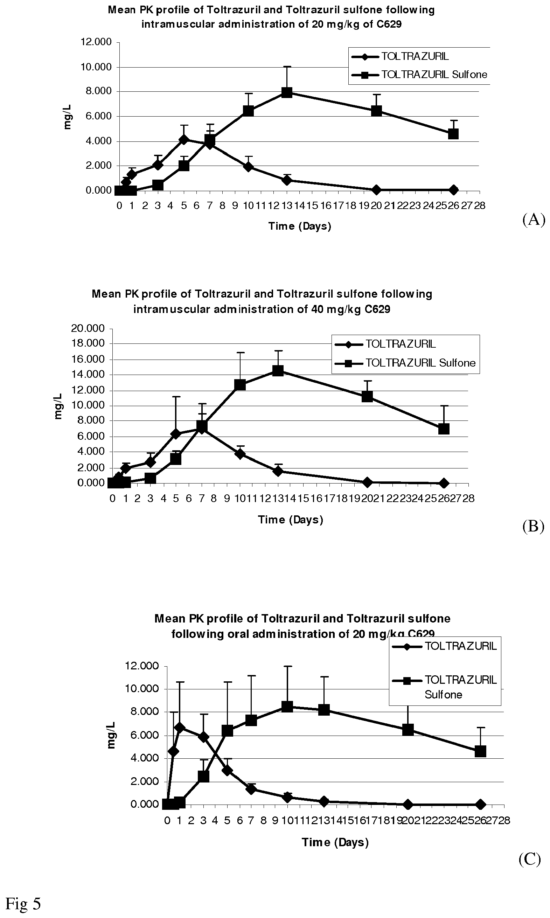

As discussed above, the inventors have found that full activity can be achieved with a single intramuscular injection of toltrazuril, and that such administration in young animals (newborn to 3 days old) can provide the required serum concentrations and release schedule of the active agent to protect the animal against infections with protozoans such as coccidia. In particular, although the intramuscular application modifies the pharmacokinetic profile of toltrazuril itself, it has now been surprisingly found that such application leads to an optimized pharmacokinetic profile of an active metabolite thereof, toltrazuril-sulfone (i.e., ponazuril). Figures 4 and 5 show that, upon intramuscular injection, a rapid and sustained serum level of toltrazuril sulfone is obtained, conferring optimal protection of young animals against coccidiosis. The invention thus allows an efficient administration of toltrazuril, even at very early stages of growth of the animals.

As discussed above, the triazine is toltrazuril.

### Intramuscular injection

An important aspect of the invention resides in the intramuscular administration route. As shown in the experimental section, the invention shows that intramuscular toltrazuril, even after a single administration, provide effective protective effect in the piglet, without the need for repeated injections or for long-term or slow release formulations. Furthermore, the invention shows intramuscular toltrazuril may be combined with further intramuscular active agents so that an effective treatment is obtained without imposing additional manipulations to the animals.

The compositions of the invention may be administered by intramuscular injection(s) using techniques and/or devices known *per se* in the art. In this regard, intramuscular injection can be performed with a syringe, a gun, a micro-needle injection device, a needle-free injection device, a pulse device, etc. In a preferred embodiment, injection is performed with a needle injector or a syringe. In another particular embodiment, injection is performed with a needle-free injection device such as a pulse needle-free system, more particularly a spring-powered, battery-powered, or compressed-gas-powered device. Specific examples of needle free technologies are described e.g., in WO2006/058426, WO2007/140610, or WO2009/111794. A preferred needle-free injection device for use in the present invention is AcuShot™ needle free technology described in the international patents WO2006/058426 and WO2007/140610.

Intramuscular injection may be made in any muscle. For livestock, such as cattle, intramuscular injection is preferably made in the area of the neck, or behind the ear, rather than in the hind limb/ham muscle or in the inguinal fold. The results presented show that intramuscular triazines exhibit potent therapeutic effect when administered in the area of the neck.

In a particular embodiment, the animal has not been exposed yet to the causative agent of coccidiosis and the method can be used to prevent or reduce infection. In another embodiment, the animal has already been exposed to the causative agent and the treatment is used to prevent or delay development of the disease and symptoms, or to reduce or cure the disease, or to avoid/limit disease spreading.

The compositions of the invention are administered by a single intramuscular injection to the pig. The results show that a single intramuscular injection is sufficient to protect a pig against coccidiosis. As illustrated, when a single intramuscular injection of toltrazuril is made between newborn and 3 days after birth, a protective effect is obtained at day 3-5, when the first disease symptoms are expected to appear.

Accordingly, the intramuscular toltrazuril of the invention is administered in newborn to 3 days old piglets, preferably between newborn to 48 hours after birth, so as to ensure a most efficient protection than an oral administration at the same time (newborn to 3 days old).

The dose of triazine may vary depending on the non-human mammal species and nature of the triazine. Conventional dosage rates from 10 to 30 mg of triazine per kg bodyweight (mg/kg) of the animal may be used. Illustrative dosages in the compositions of the invention are 10 mg, 20 mg, 30 mg, 40 mg, 50 mg or 60 mg toltrazuril/dose.

Within the context of the invention, the term "effective amount of' designates, preferably, a dose of the active agent which produces a clinical benefit in the treated animals. Particularly, an effective amount is an amount sufficient to reduce infection, disease development, or to improve the symptoms.

Preferred dosages for intramuscular toltrazuril are disclosed below, for different non-human mammal species:
- pigs: 20 mg/kg bodyweight/treatment (preferably one single administration);
- cattle: 15 mg/kg bodyweight/treatment (preferably one single administration);
- sheep: 20 mg/kg bodyweight/treatment (preferably one single administration).

In this regard, a preferred embodiment of the invention resides in a composition comprising from 10 to 30 mg toltrazuril per kg bodyweight for use in protecting a pig against coccidiosis, wherein said composition is administered by a single intramuscular injection, preferably in the neck or behind the ear.

The compositions are suspensions. The compositions may further comprise veterinary acceptable excipient(s) such as solvents, solubilizers, antioxidants, preservatives, thickeners, anti-foaming agents, etc. Suitable solvents include, without limitation, physiologically acceptable water, alcohols, esters, vegetable oils; and mixtures thereof, in isotonic solutions. Solubilizers include, e.g., polyvinylpyrrolidone. Examples of suitable antioxidants include ascorbic acid, gallic acid esters, and sulphites; and suitable preservatives include, without limitation, benzyl alcohol, n-butanol, benzalkonium chloride, benzoic acid or citric acid. Anti-foaming agents include without limitation oil carrier such as mineral oil, vegetable oil, white oil or any other oil that is insoluble in the foaming medium, silicone oil, simethicone emulsion, wax and/or hydrophobic silica such as ethylene bis stearamide (EBS), paraffinic waxes, ester waxe, silica, fatty alcohol, fatty acid soaps or esters, silicone compound, polyethylene glycol and polypropylene glycol copolymers and alkyl polyacrylates.

An example of a composition for use in the invention is a suspension comprising toltrazuril (between 10 to 30 mg toltrazuril per kg bodyweight) in water, and an anti-foaming agent.

A specific example of a suspension composition for use in the invention comprises 30 mg of toltrazuril, 3mg of docusate sodium, 100mg of polyvinylpyrrolidone, 100mg of ethanol and water qs for 1 ml.

As indicated above, the composition may comprise further active agents, such as antibiotics, anthelmintics, endectocides, anti-inflammatory agents, vitamins, and/or mineral(s) such as iron, for single, grouped, separated or sequential intramuscular injection. Preferably, the toltrazuril and the other agent(s) are combined in the same formulation for a single intramuscular injection.

In a preferred embodiment, the other agent is or comprises iron, preferably an iron complex. In parenterally injectable preparations against iron-deficiency anemia, the most common preparations accepted today comprise a combined product of ferric oxyhydroxide (or ferric hydroxide) in association with a saccharide, notably dextran or dextrin. Additional iron preparations are known, such as iron-sucrose, iron-oligosaccharide and iron-gluconate compounds.

Examples of preferred iron complexes include an aqueous colloidal solution of beta-ferric oxyhydroxide and dextran glucoheptonic acid (Gleptoferron commercialized under the trademark Gleptosil® or Ursoferran®); a ferric hydroxide with a low molecular weight dextran (commercialized under the trademark Uniferon® or Dexafer®); a ferric hydroxide with macromolecular dextran (commercialized under the trademark Ferroforte®); or a ferric compound of type I.

In a further preferred embodiment, the composition of the invention is a suspension comprising toltrazuril and an iron complex. More particularly, a specific and preferred example of a composition of the invention is a suspension comprising: toltrazuril, an iron complex, and an anti-foaming agent. A more specific example is a suspension comprising toltrazuril (preferably between 10 to 60 mg), an iron complex (preferably of beta-ferric oxyhydroxide and dextran glucoheptonic acid), water, and an anti-foaming agent.

In this regard, a further object of the invention resides in a composition comprising toltrazuril and a further active agent, for use for protecting a pig against coccidiosis, wherein said composition is administered by intramuscular injection.

Preferably, both active agents are formulated together as a suspension. In a more preferred embodiment the two active agents are administered as a single intramuscular injection.

A particular embodiment of the invention resides in a composition for use to protect pigs against coccidiosis, wherein the composition comprises toltrazuril and an iron complex, and wherein the composition is administered by a single intramuscular injection. In a more preferred embodiment, the composition comprises 10 to 30 mg/kg bodyweight triazine and a complex of iron, preferably in suspension.

### Triazine - iron combined injection

It has been proposed in the art to combine a triazine with iron, in order to treat both coccidiosis and anemia. However, such a combination raises formulation and administration problems. Indeed, the triazine is usually administered orally, while the iron compound is typically injected. EP2164196 proposed oral compositions of both agents but, at the same time, recognizes that iron compounds administered orally exhibit low bioavailability and should be administered within 8 to 10 hours after birth, leading to a separate schedule of administration for both compounds. In addition, oral administration prevents strict control of the dosage administered to each animal. Moreover, it has been disclosed that depending on the dosage, iron compound administered by injection may cause side effects.

Accordingly, there is a need in the art for an optimized triazine/iron combination treatment that is efficient and convenient. Such an optimized product would preferably contain a triazine (e.g., toltrazuril) and iron in the same formulation, suitable for combined administration, with dosages, amounts, and pharmacokinetics adapted to provide effective protection of non-human mammal such as piglets, even shortly after birth.

The invention allows an optimized combined administration of toltrazuril with iron. The invention discloses optimal dosage schedule and administration route for both compounds, to confer best protective effect. The invention further shows that, contrary to the usual knowledge, it is not stressful for one day animals to be treated by a combined intramuscular injection of toltrazuril, and iron (as complex).

The invention thus allows an efficient method for treating coccidiosis and anemia in pigs, even at very early stages of growth of the animals.

A particular object of the invention resides in an injectable composition comprising toltrazuril and iron (as complex), wherein said composition comprises, in a total volume of 0.5 to 2.0ml, 100-400mg of an iron (as complex) compound and 10-120 mg toltrazuril.

Such a composition contains the amount of toltrazuril and iron (as complex) required to obtain the minimal effective dose to ensure efficient protection. Such a composition is suitable for treating animals (e.g., piglets) of 0.40 to 5 kg, with no need for dilutions.

A preferred composition of the invention is a unitary dosage of 1.5ml comprising 37,5mg toltrazuril and 200mg of an iron compound.

As shown in the experimental section, such a product provides optimized amounts of both agents, in a suitable form for combined delivery in one single injection, with no side effect.

The compositions of the invention are formulated for injection and contain suitable excipients or vehicles such as diluents, adjuvants, stabilizers, preservatives, thickeners, anti-foaming etc.

Another object of the invention resides in an injectable composition as defined above for use in treating coccidiosis and anemia in a pig, wherein said composition is administered by a single intramuscular injection.

The invention is particularly aimed to treat young piglets, between newborn and 3 days after birth, which usually have a weight of between 0.40 to 5kg. For treating such population, there is no need to dilute the compositions, nor to weight the animal for adjustment.

The disclosure also relates to a method for determining the optimal treatment regimen for a young non-human mammal, the method comprising:
- selecting a fixed total volume (V) for the injectable composition, said volume being selected between 0.5 and 2.0ml;
- determining the volume (VI) and concentration of an iron compound source material suitable to obtain a concentration of 200mg/ml in the final composition;
- determining the concentration of a toltrazuril source material required to obtain a concentration of 20 to 50 mg/ml in the final composition when a volume V2 of such a source material is used, wherein V1+V2 = V; and
- optionally mixing the iron and toltrazuril compounds in one or several suitable excipients to prepare the formulation.

Particular emphasis may be placed on pigs in all species, subspecies and breeds.

Further aspects and advantages of the invention will be disclosed in the following illustrative experimental section.

### Examples

### Example A - Local and general tolerance of a single intramuscular injection of toltrazuril in 3-day old piglets.

### Protocol

This target animal safety and efficacy study was conducted in a farm with known history of cocciodiosis. The main objective was to study the safety and efficacy of intramuscular injection of toltrazuril in comparison to the standard oral application.
Group 1: 8 piglets 3 days old were injected by a single intramuscular dose of toltrazuril (Dose = 20 mg toltrazuril per kg bodyweight equivalent to 0,4mL/kg bodyweight) on SD0.
Group 2: 8 piglets 3 days old were injected by one intramuscular dose of toltrazuril (60 mg toltrazuril per kg bodyweight equivalent to 1,2mL/Kg bodyweight) on SD0
Group 3: 8 piglets 3 days old were dosed orally with the standard commercial formulation of toltrazuril Cevazuril® (Dose = 20 mg toltrazuril per kg bodyweight equivalent to 0,4 mL/Kg bodyweight) on SD0
Group 4: 12 piglets 3 days old, left untreated.

All four (4) groups of piglets were injected on SD0 with iron (Gleptosil®) at the dose rate of 1 ml per piglet.

### Results

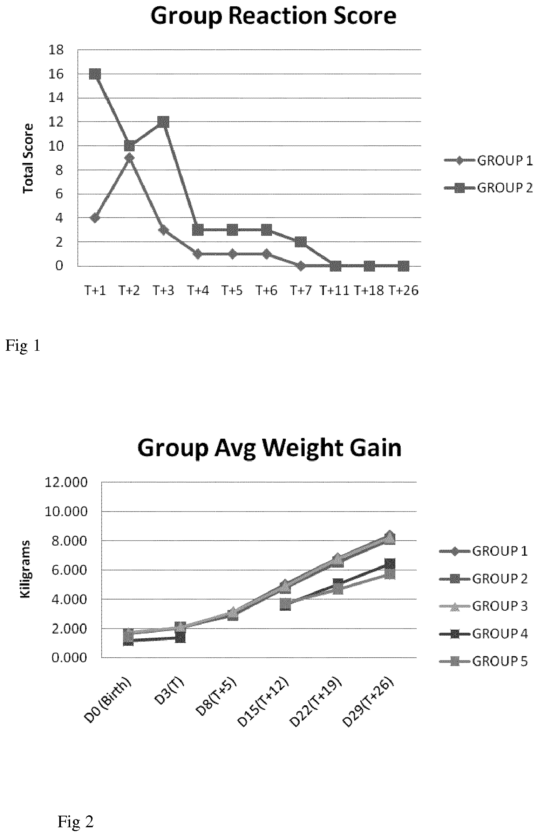

The local and general tolerances, as well as bodyweight development were assessed. The results are presented Fig 1 and Fig 2 and can be summarized as follows:
- Intramuscular doses up to 60 mg/kg was well tolerated
- No pain, limited local reaction (Oedema) in some piglets injected with 1.2 mL/Kg bodyweight, disappearing within less than 1 week post injection
- The bodyweight development was normal in treated piglets.
- The intramuscularly treated piglets gained more than control non treated piglets (+2 kg at SD0+29days)

### Example B - Pharmacokinetics, safety and anti-coccidial efficacy of intramuscular toltrazuril in 2 day-old piglets when applied once.

### Protocol

Group 1: 10 piglets 2 days old were injected with once intramuscularly dose of toltrazuril C629 (Dose = 20 mg toltrazuril per kg bodyweight - equivalent to 0,4mL/Kg bodyweight) on SD0
Group 2: 11 piglets 2 days old were injected with once intramuscularly dose of toltrazuril C629 (Dose = 40 mg toltrazuril per kg bodyweight a- equivalent to 0,8 mL/kg bodyweight) on SD0
Group 3: 9 piglets 2 days old were dosed orally with the standard commercial formulation of toltrazuril Cevazuril® (Dose = 20 mg toltrazuril per kg bodyweight -equivalent to 0,4 mL/Kg bodyweight) on SD0.
Group 4: 8 piglets, left untreated.

All four (4) groups of piglets were injected on SD0 with iron injection (Gleptosil®) at the dose rate of 1 ml per piglet. 3 days after treatment (on SD3), the piglets were orally challenged with a characterised strain of *Isospora suis.*

The following study parameters were used to assess the efficacy of the test products:
- Local and general tolerance (for Groups 1 and 2)
- Occurrence of diarrhoea and faecal consistency
- Oocyst excretion
- Bodyweight development
- Serum concentrations of toltrazuril and its metabolite toltrazuril sulfone (e.g ponazuril) in Group 1, 2 and 3.

### Results

The results are presented Fig 3 to 5 and in the following Table 1. They can be summarized as follows.
- All the tested doses were well tolerated
- No oocyst excretion was observed in treated animals and the fecal consistency was unchanged in treated piglets (G1, G2 and G3).
- The animals infected and treated orally or intramuscularly (G1, G2 G3) gained more weight than the control animals (G4).
- Toltrazuril is well absorbed after intramuscular application.
- The toltrazuril sulfone (e.g ponazuril) kinetics is not statistically different in animals treated orally (G3) or intramuscularly (G1 and G2)

**Table 1**

| Pharmacokinetic Parameters | Group 1 (i.m. 20 mg/kg) | Group 2 (i.m. 40 mg/kg) |
|---|---|---|
| Tmax (h) | 157 | 171 |
| Cmax (mg/L) | 4025 | 7293 |
| Cmax/Dose | 193 | 182 |
| AUCinf (mg*h/L) | 854714 | 1362678 |
| AUCinf/Dose | 39551 | 34067 |

## Claims

1. A composition comprising between 10 and 30 mg per kg bodyweight toltrazuril for use in the preventive treatment of an infection with coccidia of the genus *Isospora* in a newborn to 3 days old pig, wherein said composition is administered by a single intramuscular injection and wherein the composition is a suspension.

2. The composition for use of claim 1, wherein the composition is administered 0-48 hours after birth.

3. The composition for use of any one of claims 1 or 2, wherein the composition is administered in a muscle of the neck or behind the ear.

4. The composition for use of any one of the previous claims, which is an injectable composition comprising toltrazuril and an iron complex, wherein said composition comprises, in a total volume of 0.5 to 2.0 ml, 100-400 mg of an iron complex compound and 10-120 mg toltrazuril.

5. The composition for use of claim 4, wherein the iron complex is gleptoferron.

6. The composition for use of any one of the preceding claims, which is an aqueous and/or alcoholic suspension.

7. The composition for use of any one of the preceding claims, which further comprises one or several excipients.

## Patentansprüche

1. Zusammensetzung, umfassend zwischen 10 und 30 mg pro Kilogramm Körpergewicht Toltrazuril zur Verwendung in der präventiven Behandlung einer Infektion mit Kokzidien der Gattung *Isospora* in einem neugeborenen bis drei Tage alten Schwein, wobei die Zusammensetzung durch eine einzige intramuskuläre Injektion verabreicht wird und wobei die Zusammensetzung eine Suspension ist.

2. Zusammensetzung nach Anspruch 1, wobei die Zusammensetzung innerhalb von 0 - 48 Stunden nach der Geburt verabreicht wird.

3. Zusammensetzung zur Verwendung nach einem der Ansprüche 1 oder 2, wobei die Zusammensetzung in einen Muskel am Nacken oder hinter dem Ohr verabreicht wird.

4. Zusammensetzung zur Verwendung nach einem der vorstehenden Ansprüche, die eine injizierbare Zusammensetzung ist, die Toltrazuril und einen Eisenkomplex umfasst, wobei die Zusammensetzung 100 - 400 mg einer Eisenkomplex-Zusammensetzung und 10 - 120 mg Toltrazuril in einem Gesamtvolumen von zwischen 0,5 und 2,0 ml umfasst.

5. Zusammensetzung zur Verwendung nach Anspruch 4, wobei der Eisenkomplex Gleptoferron ist.

6. Zusammensetzung nach einem der vorstehenden Ansprüche, welche eine wässrige und/oder alkoholische Suspension ist.

7. Zusammensetzung nach einem der vorstehenden Ansprüche, welche des weiteren einen oder mehrere Hilfsstoffe umfasst.

## Revendications

1. Composition comprenant entre 10 et 30 mg par kg de poids corporel de toltrazuril pour utilisation dans le traitement préventif d'une infection par coccidies du genre *Isospora* chez un porc nouveau-né jusqu'à 3 jours de vie, ladite composition étant administrée par une injection intramusculaire unique et ladite composition étant une suspension.

2. Composition pour utilisation selon la revendication 1, ladite composition étant administrée entre 0 et 48 heures après la naissance.

3. Composition pour utilisation selon l'une des revendications 1 ou 2, ladite composition étant administrée dans un muscle du cou ou derrière l'oreille.

4. Composition pour utilisation selon l'une quelconque des revendications précédentes, qui est une composition injectable comprenant du toltrazuril et un complexe de fer, ladite composition comprenant, dans un volume total de 0.5 à 2.0 ml, 100-400 mg d'un composé de complexe de fer et 10-120 mg de toltrazuril.

5. Composition pour utilisation selon la revendication 4, dans laquelle le complexe de fer est le gleptoferron.

6. Composition pour utilisation selon l'une des revendications précédentes, qui est une suspension aqueuse et/ou alcoolique.

7. Composition pour utilisation selon l'une des revendications précédentes, comprenant en outre un ou plusieurs excipients.
